# EUROPEAN PATENT SPECIFICATION

(11) **EP 2 098 199 B1**
(45) Date of publication and mention of the grant of the patent: **05.01.2011**
(21) Application number: 08004004.1
(22) Date of filing: 04.03.2008
(51) Int. Cl.: A61F 9/06

(54) **Welding mask**
Schweißmaske
Masque de soudage

(43) Date of publication of application: 09.09.2009
(73) Proprietor: Polison Corporation, Kaohsiung City (TW)
(72) Inventor: Chiang, Hui-Min, Kaohsiung City (TW)
(74) Representative: Munk, Ludwig

(56) References cited:
- WO-A-00/59421
- GB-A- 2 202 644
- US-A- 3 257 667
- US-A- 3 444 561
- US-A- 5 140 707

## Description

The invention relates to a welding mask, more particularly to a welding mask that can be assembled without damaging a lens thereof.

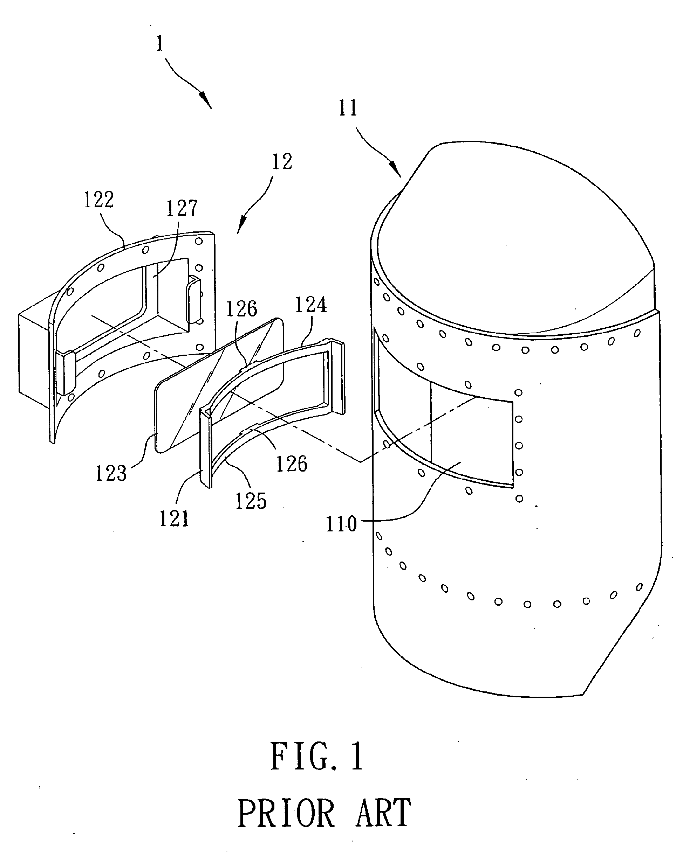

Figure 1 illustrates a conventional welding mask 1 that includes a mask body 11 and screen assembly 12. The mask body 11 has a viewing window 110. The screen assembly 12 serves to cover the viewing window 110. In particular, the screen assembly 12 includes first and second frames 121, 122 and a lens 123. The first frame 121 is mounted on the mask body 11, and includes opposite top and bottom frame parts 124, 125, each of which is formed with a protrusion 126. The second frame 122 is connected detachably to the first frame 121, and includes a flange 127 that extends radially and inwardly therefrom. The lens 123 is sandwiched between the protrusions 126 and the flange 127.

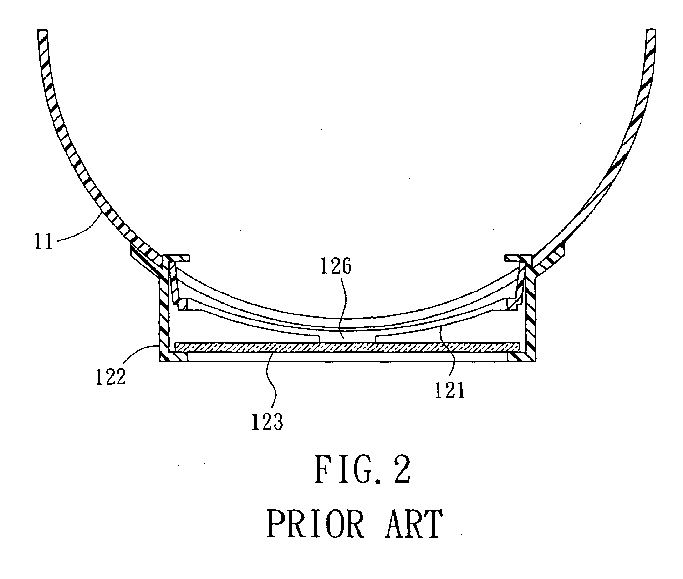

The aforementioned conventional welding mask 1 is disadvantageous in that, during assembly, the lens 123 is easily damaged, i.e., scratched or broken, due to urging forces applied thereon by the protrusions 126 when the second frame 122 is connected to the first frame 121, as best shown in Figure 2.

In order to solve the above-described problem, it has been proposed to use the welding mask disclosed in U.K. Patent Application Publication No. GB2202644. The welding mask includes a mask body that has a viewing window, a first frame that is mounted on the mask body, a second frame that is connected detachably to the first frame, a lens spring assembly that is disposed between the first and second frames, and lens that is disposed between the lens spring assembly and the first frame. However, since the lens spring assembly of the welding mask is a separate part, the number of parts thereof is therefore increased. This results in inconvenience during assembly of the welding mask, and thus results in high manufacturing costs thereof. Moreover, the welding mask, like the conventional welding mask 1, has to be removed or lifted up whenever the welder wants to have a clearer view.

Therefore, the object of the present invention is to provide a welding mask that can overcome the aforesaid drawbacks of the prior art.

According to the present invention, a welding mask comprises a mask body and a screen assembly. The mask body has a viewing window. The screen assembly serves to cover the viewing window, and includes first and second frames, an elastic piece, a lens, third and fourth frames, a second elastic piece, and a second lens. The first frame is mounted on the mask body. The second frame is connected detachably to the first frame. The elastic piece is provided on the second frame. The lens is sandwiched between the elastic piece and the first frame. The third frame is connected pivotably to the mask body. The fourth frame is connected detachably to the third frame. The second elastic piece is provided on the fourth frame. The second lens is sandwiched between the second elastic piece and the third frame.

Other features and advantages of the present invention will become apparent in the following detailed description of the preferred embodiments with reference to the accompanying drawings, of which:
Figure 1 is an exploded perspective view of a conventional welding mask;
Figure 2 is a schematic sectional view of the conventional welding mask;
Figure 3 is an exploded perspective view of the first preferred embodiment of a welding mask according to the present invention;
Figure 4 is a sectional view of the first preferred embodiment;
Figure 5 is an exploded perspective view illustrating a screen assembly of the second preferred embodiment of a welding mask according to this invention; and
Figure 6 is a perspective view to illustrate a state where a second lens uncovers a lens of the second preferred embodiment.

Before the present invention is described in greater detail, it should be noted that like elements are denoted by the same reference numerals throughout the disclosure.

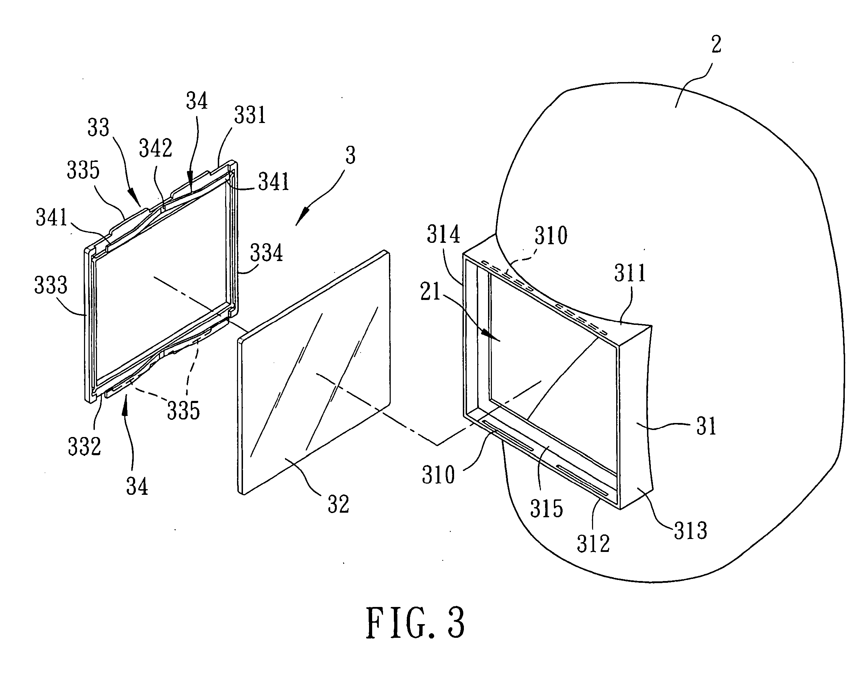

Referring to Figure 3, the first preferred embodiment of a welding mask according to this invention is shown to include a mask body 2 and screen assembly 3.

The welding mask of this invention protects the eyes of a welder from flying debris and an intense light generated during a welding operation, in a manner that will be described hereinafter.

The mask body 2 has a viewing window 21. In this embodiment, the viewing window 21 may be sized in such a manner as to maximize a viewing angle or minimize the cost of the screen assembly 3.

The screen assembly 3 serves to cover the viewing window 21. In particular, the screen assembly 3 includes first and second frames 31, 33, a lens 32, and a pair of elastic pieces 34.

The first frame 31 is mounted on the mask body 2, and includes opposite top and bottom frame parts 311, 312 and opposite left and right frame parts 313, 314, and a flange 315 that extends radially and inwardly therefrom.

The second frame 33 is connected detachably to the first frame 31. In particular, each of the top and bottom frame parts 311, 312 of the first frame 31 is formed with a pair of engaging grooves 310. The second frame 33 includes opposite top and bottom frame parts 331, 332 and opposite left and right frame parts 333, 334. The top frame part 331 of the second frame 33 is formed with a pair of engaging protrusions 335, each of which extends into a respective one of the engaging grooves 310 in the top frame part 311 of the first frame 31. The bottom frame part 332 of the second frame 33 is formed with a pair of engaging protrusions 335, each of which extends into a respective one of the engaging grooves 310 in the bottom frame part 312 of the first frame 31.

Although the first and second frames 31, 33 of the screen assembly 3 of the welding mask of this invention are exemplified using two pairs of engaging grooves and two pairs of engaging protrusions, respectively, it should be apparent to those skilled in the art that the number of engaging grooves and engaging protrusions may be increased or decreased as required.

Each of the elastic pieces 34 is provided on a respective one of the top and bottom frame parts 331, 332 of the second frame 33. In this embodiment, each of the elastic pieces 34 is in the form of a spring arm, and has opposite free end portions 341, and a fixed portion 342 disposed between the free end portions 341 thereof and connected to a respective one of the top and bottom frame parts 331, 332 of the second frame 33.

It is noted that the free end portions 341 of each of the elastic pieces 34 are spaced apart from the second frame 33 when the second frame 33 is detached from the first frame 31.

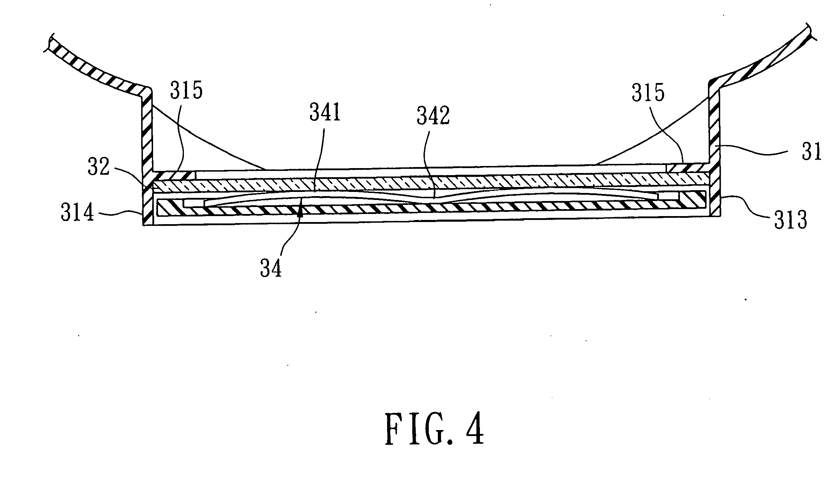

The lens 32 has a generally rectangular shape, is surrounded by the top and bottom frame parts 311, 312 and the left and right frame parts 313, 314 of the first frame 31, and is sandwiched between the free end portions 341 of the elastic pieces 34 and the flange 315 of the first frame 31, as best shown in Figure 4. In this embodiment, the lens 32 is an obscured lens.

Although the second frame 33 of the screen assembly 3 of this invention is exemplified using a pair of elastic pieces, it should be apparent to those skilled in the art that the number of elastic pieces may be increased or decreased as required. For example, the screen assembly 3 may further include another pair of elastic pieces that are provided on the left and right frame parts 333, 334 of the second frame 33, respectively.

During assembly, the lens 32 is first disposed such that the lens 32 is surrounded by the top and bottom frame parts 311, 312 and left and right frame parts 313, 314 of the first frame 31 and abuts against the flange 315 of the first frame 31. Then, the second frame 33 is disposed such that each of the elastic pieces 34 abuts against the lens 32. Thereafter, the second frame 33 is connected to the first frame 31. Each of the elastic pieces 34 absorbs an urging force applied on the lens 32 when the second frame 33 is connected to the first frame 31. As a result, scratching or breaking of the lens 32 is prevented.

When it is desired to replace the lens 32, the second frame 33 is first detached from the first frame 31 by simply deforming the second frame 33 such that each of the engaging protrusions 335 extends out of the respective one of the engaging grooves 310. Once the lens 32 is replaced, the second frame 33 is connected back to the first frame 31.

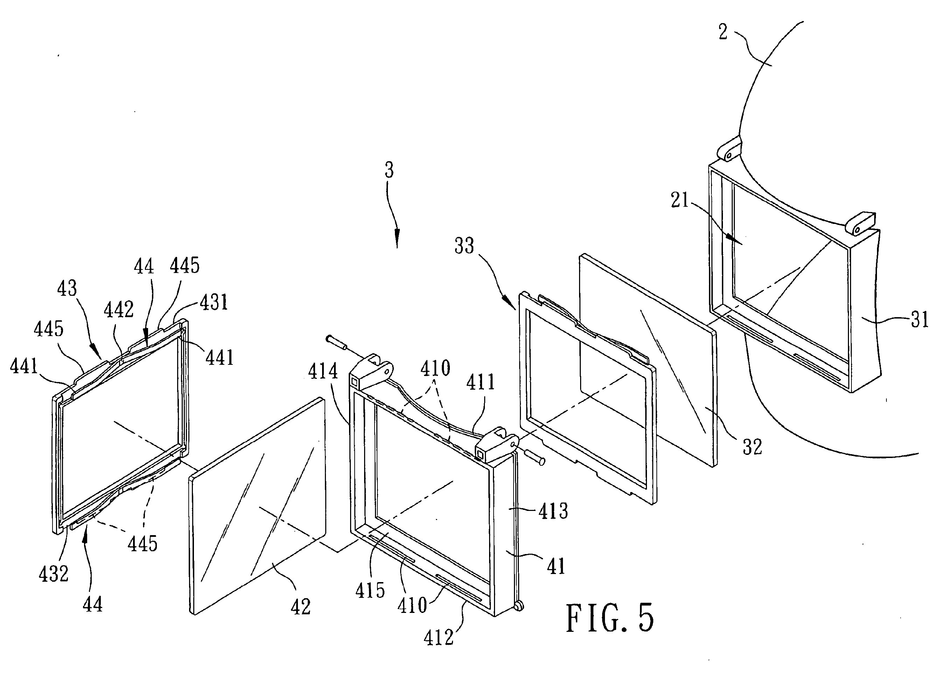

Figure 5 illustrates the second preferred embodiment of a welding mask according to this invention. When compared to the previous embodiment, the screen assembly 3 further includes third and fourth frames 41, 43, a second lens 42, and a pair of second elastic pieces 44.

The third frame 41 has a structure identical to the first frame 31 and is connected pivotably to the mask body 2.

The fourth frame 43 has a structure identical to the second frame 33, and is connected detachably to the third frame 41. That is, each of engaging protrusions 445 of the fourth frame 43 extends into a respective one of engaging grooves 410 in the third frame 41.

Each of the second elastic pieces 44 has a structure identical to the elastic pieces 34 and is provided on a respective one of top and bottom frame parts 431, 432 of the fourth frame 43. That is, each of the elastic pieces 44 is in the form of a spring arm, and has opposite free end portions 441, and a fixed portion 442 disposed between the free end portions 441 thereof and connected to a respective one of the top and bottom frame parts 431, 432 of the fourth frame 43.

It is noted that the free end portions 441 of each of the elastic pieces 44 are spaced apart from the fourth frame 43 when the fourth frame 43 is detached from the third frame 41.

The second lens 42 has a generally rectangular shape, is surrounded by top and bottom frame parts 411, 412 and the left and right frame parts 413, 414 of the third frame 41, and is sandwiched between the free end portions 441 of the elastic pieces 44 and a flange 415 of the third frame 41.

It is noted that, in this embodiment, the lens 32 is a transparent lens and the second lens 42 is an obscured lens.

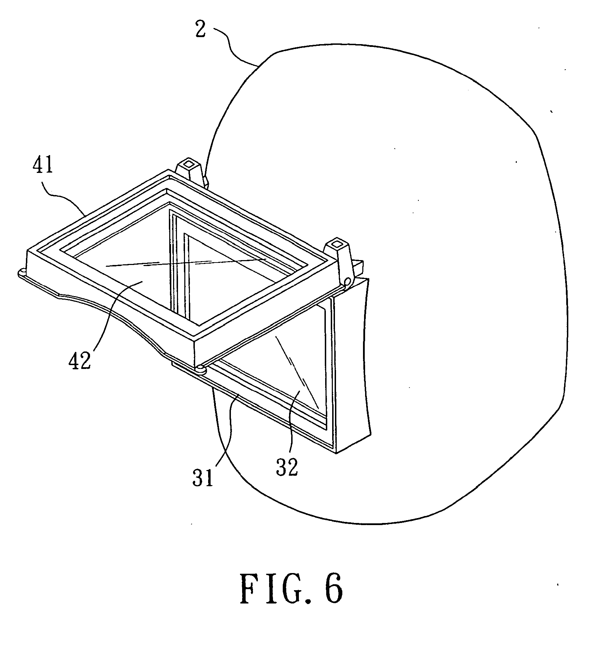

The third frame 41 is pivotable relative to the mask body 2 between a first position, where the second lens 42 covers the lens 32, and a second position, where the second lens 42 uncovers the lens 32, as best shown in Figure 6.

In use, during a welding operation, the third frame 41 may be disposed at the first position to thereby protect the eyes of the welder from an intense light generated during the welding operation. When not performing a welding operation, however, the third frame 41 may be disposed at the second position to give the welder a clearer view without having to remove or lift up the welding mask of this invention.

## Claims

1. A welding mask, comprising:
a mask body (2) having a viewing window (21); and
a screen assembly (3) for covering said viewing window (21), said screen assembly (3) including
a first frame (31) mounted on said mask body (2),
a second frame (33) connected detachably to said first frame (31),
an elastic piece (34) provided on said second frame (33), and
a lens (32) sandwiched between said elastic piece (34) and said first frame (31), **characterized in that** said screen assembly further includes
a third frame (41) connected pivotably to said mask body (2),
a fourth frame (43) connected detachably to said third frame (41),
a second elastic piece (44) provided on said fourth frame (43), and
a second lens (42) sandwiched between said second elastic piece (44) and said third frame (41).

2. The welding mask as claimed in Claim 1, **characterized in that** said elastic piece (34) is in the form of a spring arm.

3. The welding mask as claimed in Claim 1, **characterized in that** said elastic piece (34) has opposite free end portions (341) spaced apart from said second frame (33) when said second frame (33) is detached from said first frame (31), and a fixed portion (342) disposed between said free end portions (341) thereof and connected to said second frame (33).

4. The welding mask as claimed in Claim 1, **characterized in that** said first frame (31) is formed with an engaging groove (310), and said second frame (33) is formed with an engaging protrusion (335) that extends into said engaging groove (310).

5. The welding mask as claimed in Claim 1, **characterized in that** said second elastic piece (44) is in the form of a spring arm.

6. The weldingmask as claimed in Claim 1, **characterized in that** said second elastic piece (44) has opposite free end portions (441) spaced apart from said fourth frame (43) when said fourth frame (43) is detached from said third frame (41), and a fixed portion (442) disposed between said free end portions (441) thereof and connected to said fourth frame (43).

7. The welding mask as claimed in Claim 1, **characterized in that** said third frame (41) is formed with an engaging groove (410), and said fourth frame (43) is formed with an engaging protrusion (445) that extends into said engaging groove (410).

## Patentansprüche

1. Schweißmaske, umfassend:
einen Maskenkörper (2) mit einem Sichtfenster (21); und eine Schirmanordnung (3) zur Abdeckung des Sichtfensters (21), wobei die Schirmanordnung (3) einschließt:
einen ersten Rahmen (31), der auf dem Maskenkörper (2) montiert ist,
einen zweiten Rahmen (33), der lösbar mit dem ersten Rahmen (31) verbunden ist,
ein elastisches Teil (34), das an dem zweiten Rahmen (33) vorgesehen ist, und
eine Linse (32), die zwischen dem elastischen Teil (34) und dem ersten Rahmen (31) angeordnet ist, **dadurch gekennzeichnet, dass** die Schirmanordnung außerdem einschließt:
einen dritten Rahmen (41), der schwenkbar an dem Maskenkörper (2) angebracht ist,
einen vierten Rahmen (43), der lösbar mit dem dritten Rahmen (41) verbunden ist,
ein zweites elastisches Teil (44), das an dem vierten Rahmen (43) vorgesehen ist, und
eine zweite Linse (42), die zwischen dem zweiten elastischen Teil (44) und dem dritten Rahmen (41) angeordnet ist.

2. Schweißmaske nach Anspruch 1, **dadurch gekennzeichnet, dass** das elastische Teil (34) in Form eines Federarmes vorliegt.

3. Schweißmaske nach Anspruch 1, **dadurch gekennzeichnet, dass** das elastische Teil (34) gegenüberliegende freie Endabschnitte (341) aufweist, die von dem zweiten Rahmen (33) beabstandet sind, wenn der zweite Rahmen (33) von dem ersten Rahmen (31) gelöst ist, sowie einen fixierten Abschnitt (342), der zwischen den freien Endabschnitten (341) desselben angeordnet und mit dem zweiten Rahmen (33) verbunden ist.

4. Schweißmaske nach Anspruch 1, **dadurch gekennzeichnet, dass** der erste Rahmen (31) mit einer Rastnut (310) ausgebildet ist und der zweite Rahmen (33) mit einem Rastvorsprung (335) ausgebildet ist, der sich in die Rastnut (310) erstreckt.

5. Schweißmaske nach Anspruch 1, **dadurch gekennzeichnet, dass** das zweite elastische Teil (44) in Form eines Federarmes vorliegt.

6. Schweißmaske nach Anspruch 1, **dadurch gekennzeichnet, dass** das zweite elastische Teil (44) gegenüberliegende freie Endabschnitte (441) aufweist, die von dem vierten Rahmen (43) beabstandet sind, wenn der vierte Rahmen (43) von dem dritten Rahmen (41) gelöst ist, sowie einen fixierten Abschnitt (442), der zwischen den freien Endabschnitten (441) desselben angeordnet und mit dem vierten Rahmen (43) verbunden ist.

7. Schweißmaske nach Anspruch 1, **dadurch gekennzeichnet, dass** der dritte Rahmen (41) mit einer Rastnut (410) ausgebildet ist und der vierte Rahmen (43) mit einem Rastvorsprung (445) ausgebildet ist, der sich in die Rastnut (410) erstreckt.

## Revendications

1. Un masque de soudage comprenant :
un corps de masque (2) avec un hublot (21) ; et un assemblage d'écran (3) pour couvrir ledit hublot (21), ledit assemblage d'écran (3) comprenant :
un premier cadre (31) monté sur ledit corps de masque (2),
un deuxième cadre (33) raccordé de façon amovible au premier cadre (31),
une pièce élastique (34) se trouvant sur ledit deuxième cadre (33), et
une lentille (32) prise en sandwich entre ladite pièce élastique (34) et ledit premier cadre (31), **caractérisé en ce que** ledit assemblage d'écran inclue également
un troisième cadre (41) raccordé de manière à pouvoir pivoter au corps du masque (2),
un quatrième cadre (43) raccordé de façon amovible au troisième cadre (41),
une deuxième pièce élastique (44) se trouvant sur ledit quatrième cadre (43), et
une deuxième lentille (42) prise en sandwich entre ladite deuxième pièce élastique (44) et ledit troisième cadre (41).

2. Le masque de soudage comme revendiqué dans la revendication 1, **caractérisé en ce que** ladite pièce élastique (34) est en forme de bras à ressort.

3. Le masque de soudage comme revendiqué dans la revendication 1, **caractérisé en ce que** ladite pièce élastique (34) a des portions finales libres opposées (341) espacées dudit deuxième cadre (33) lorsque ledit deuxième cadre (33) est détaché dudit premier cadre (31), et une portion fixe (342) disposée entre lesdites portions finales libres (341) et raccordée au deuxième cadre (33).

4. Le masque de soudage comme revendiqué dans la revendication 1, **caractérisé en ce que** ledit premier cadre (31) est formé par une rainure d'enclenchement (310), et ledit deuxième cadre (33) est formé par une protubérance d'enclenchement (335) qui s'étend dans ladite rainure d'enclenchement (310).

5. Le masque de soudage comme revendiqué dans la revendication 1, **caractérisé en ce que** ladite deuxième pièce élastique (44) est en forme de bras à ressort.

6. Le masque de soudage comme revendiqué dans la revendication 1, **caractérisé en ce que** ladite deuxième pièce élastique (44) a des portions finales libres opposées (441) espacées dudit quatrième cadre (43) lorsque ledit quatrième cadre (43) se détache dudit troisième cadre (41), et une portion fixe (442) disposée entre lesdites portions finales libres (441) et raccordée au quatrième cadre (43).

7. Le masque de soudage comme revendiqué dans la revendication 1, **caractérisé en ce que** ledit troisième cadre (41) est formé par une rainure d'enclenchement (410), et ledit quatrième cadre (43) est formé par une protubérance d'enclenchement (445) qui s'étend dans ladite rainure d'enclenchement (410).
